# EUROPEAN PATENT SPECIFICATION

(11) **EP 1 625 854 B1**
(45) Date of publication and mention of the grant of the patent: **15.10.2008**
(21) Application number: 04018855.9
(22) Date of filing: 09.08.2004
(51) Int. Cl.: A61K 47/48, A61P 29/00, A61P 35/00, A61P 37/00

(54) **Albumin conjugates containing a glucuronic linker**
Albuminkonjugate, die eine Glucuron-Verknüpfung enthalten
Conjugés d'albumine contenant un lien glucuronique

(43) Date of publication of application: 15.02.2006
(73) Proprietor: Deutsches Krebsforschungszentrum Stiftung des öffentlichen Rechts, 69120 Heidelberg (DE); Ruprecht-Karls-Universität Heidelberg, 69117 Heidelberg (DE)
(72) Inventor: Frei, Eva, 69120 Heidelberg (DE); Schrenk, Hans-Hermann, 67378 Zeiskam (DE); Fiehn, Christoph, 69123 Heidelberg (DE); Kremer, Paul, 69221 Dossenheim (DE); Breuer, Andrea, 69226 Nussloch (DE)
(74) Representative: Schüssler, Andrea

(56) References cited:
- EP-A- 0 482 540
- EP-A- 0 721 956
- WO-A-93/22334
- WO-A-94/15947
- PATENT ABSTRACTS OF JAPAN vol. 009, no. 192 (C-296), 8 August 1985 (1985-08-08) & JP 60 061596 A (EIKEN KAGAKU KK), 9 April 1985 (1985-04-09)
- PATENT ABSTRACTS OF JAPAN vol. 007, no. 113 (P-197), 18 May 1983 (1983-05-18) & JP 58 033162 A (EIKEN KAGAKU KK), 26 February 1983 (1983-02-26)
- PATENT ABSTRACTS OF JAPAN vol. 007, no. 105 (P-195), 7 May 1983 (1983-05-07) & JP 58 028664 A (EIKEN KAGAKU KK), 19 February 1983 (1983-02-19)
- HAEBERLIN B. ET AL: "In vitro evaluation of dexamethasone-b-D-glucoronide for colon-specific drug delivery" PHARMACEUTICAL RESEARCH, vol. 10, 1993, pages 1553-1562, XP008047216
- PALOMINO E. ET AL: "Carbohydrate prodrugs:potential use for in situ activation and drug delivery" DRUGS OF THE FUTURE, vol. 16, 1991, pages 1029-1037, XP008047254

## Description

The present invention relates to a pharmaceutical composition comprising a conjugate comprising (a) a pharmacolocically active drug, preferably a corticosteroid, (b) a glucuronic acid and (c) an albumin. The present invention also relates to uses of said conjugate for the treatment/prevention of particular diseases, preferably inflammatory diseases and tumor diseases.

Rheumatic diseases are generally treated by administration of different kinds of steroids. This kind of therapy does not cure the disease but merely alleviates the symptoms of the disease. Thus, a permanent application of the drugs is required. Since the steroids exhibit their biological effects not only in the target tissue, i.e. the inflamed tissue, but also in many other organs severe undesired side effects are observed, like cushing syndrome, osteoporosis etc. It is apparent that therapy by use of an agent which accumulates within the inflamed tissue would lead to a reduction of these side effects and, thus, would be advantageous for the patient. However, so far no convincing solutions of this problem could be provided.

Prior art documents JP 60 061596 A (EIKEN KAGAKU KK), JP 58 033162 A (EIKEN KAGAKU KK) and JP 58 028664 A (EIKEN KAGAKU KK) disclose a conjugate comprising a glucuronide of 6-oxoestradiol and albumin which is used as an antigen for producing antibodies for the determination of estradiol-3-sulfate-17β-glucuronide by immunochemical methods.

WO 94/15947 A (AKTIEBOLAGET ASTRA) discloses conjugates comprising a glucocorticosteroid (GCS) chemically bound to a sugar for colon- or ileum-specific delivery of GCS.

EP-A-0 482 540 (LABORATORI BALDACCI SPA) discloses conjugates comprising a corticosteroid linked to human albumin.

Therefore, it is the object of the present invention to provide a means which overcomes the disadvantages of the drugs of the prior art for treatment of several diseases (preferably inflammation diseases), i.e. which accumulates within the respective diseased tissue.

According to the invention this is achieved by the subject matters defined in the claims, i.e., by provision of a drug conjugate which accumulates within the (inflamed) tissue with the drug being released in the target organ in an active form. This conjugate comprises (a) a corticosteroid, (b) a glucuronic acid and (c) albumin. In preferred embodiments, such as in the experiments leading to the present invention, corticosteroids such as hydrocortisone, prednisolone, dexamethasone were linked via a β-glycosidic linkage with a D-glucuronic acid at position 21 of the steroid and position 1 of the D-glucuronic acid to form a glucuronide. This compound was linked by use of the Bolton-Hunter method as an acid amide via the carboxylic acid of the glucuronide to a lysine residue of human serum albumin (HSA) in a molar ratio of about 1:1.

Albumin and the albumin conjugates are introduced via endocytosis into tumor cells and fibroblasts in the case of inflammation and are digested by lysosomal proteases. In the same cell compartment, i.e., the lysosome, glucuronidases are localized which are capable of cleaving off the active agent from the conjugate. In the case of steroids, in particular corticosteroids, the released lipophil steroid exits the lysosome by diffusion, binds to the steroid receptor which is present in cytoplasm and exerts its biological effects. Alternatively, the conjugate can already be cleaved in the extracellular space (synovial fluid, tumor interstitial space) by secreted proteases and glucuronidases. This kind of conjugation results in an accumulation of the drug within the desired tissue, e.g., inflamed tissue and the tumor, respectively, compared to normal tissue. Accordingly, therapy by use of the conjugate of the present invention, i.e., albumin-mediated accumulation (drug targeting concept based therapy) is characterized by the following advantages: lower systemic side effects, increased half-life within the body, fewer application intervals, improvement of the quality of life of the patient.

### Brief description of the drawings

Figure 1: Formula of (A) prednisolone-21-β-D-glucuronide and (B) dexamethasone-21-β-D-glucuronide
Figure 2: Formula of (A) hydrocortisone-21-β-D-glucuronide-HSA, (B) dexamethasone-21-β-D-glucuronide-HSA and (C) prednisolone-21-β-D-glucuronide-HSA
Figure 3: Determination of the biological effect of HCG-HSA in cell culture
   See Example 2 for details
Figure 4: Release of a fluorescent aglycon from MUG-HSA in human synovial fibroblasts
   See Example 2 for details

Accordingly, the present invention provides a conjugate comprising (a) a corticosteroid (b) a glucuronic acid and (c) an albumin.

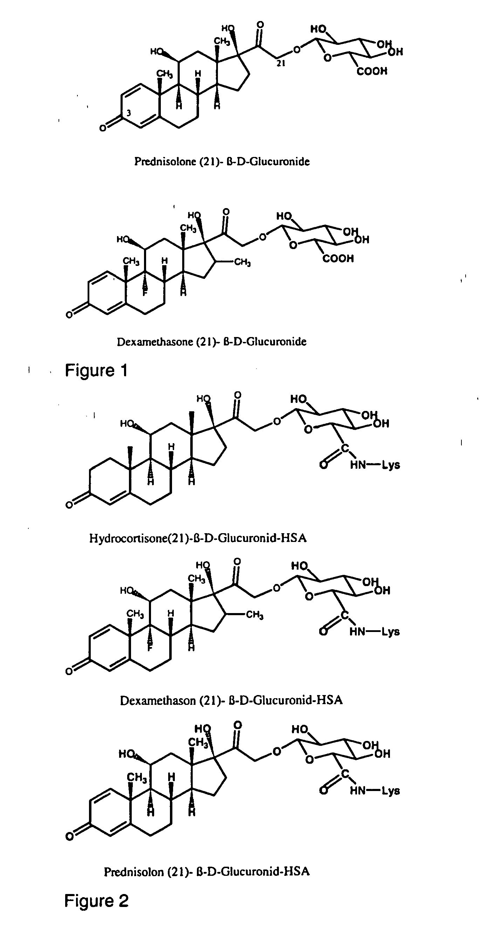

The coupling of the constituents of the conjugate is made by covalent chemical binding. Methods for preparing the components of the conjugates of the present invention and for coupling are, e.g., disclosed in Haeberlein, B. et al., Pharmac. Res. 10 (1993), 1553 (adapted for the glucuronide synthesis). Preferably, the conjugate of the present invention has the following structure: (a)-(b)-(c) (c.f. Fig. 2).

The term "pharmacologically active drug" in this context refers to any drug that contains a hydroxy group which can be conjugated with glucuronic acid, which upon a cleavage liberates the active drug and is suitable for therapy/prevention of diseases characterized by cells that can take up albumin or albumin conjugates via endocytosis, e.g., tumor cells or fibroblasts of inflamed tissue. Suitable drugs are corticosteroids (e.g., dexamethasone, hydrocortisone, prednisolone).

The term "glucuronide" as used herein refers to any compound which is produced by linking a specific position of glucuronic acid with an aglycon (e.g. active drug). The glucuronides are characterized by a α- or β-linkage of preferably the chiral C1 of glucuronic acid. The drug may be cleaved off from the glucuronide to which it is covalently coupled by a glucuronidase being present in the body. Preferably, the glucuronic acid is covalently coupled to the drug at its position 1.

The term "albumin" as used herein refers to any albumin that can be taken up by cells, preferably human tumor cells or fibroblasts of inflamed tissue, via endocytosis and digested by proteases. A preferred albumin is human serum albumin (HSA).

For the purposes of the present invention, the glucuronic acid has to be coupled to the drug in such a way, that after cleavage by a glucuronidase the drug, e.g., the corticosteroid, is present in its biologically active form. The person skilled in the art can determine suitable kinds of covalent linkage by routine experimentation and assay the biological activity of the drug after cleavage according to the methods described in the present

### examples.

In a preferred embodiment of the conjugate of the present invention, the glucuronic acid is linked at position 21 of the steroid.

The glucuronide can be coupled to any residue of the albumin as long as the transport of the conjugate by endocytosis is not hampered or inhibited. Preferably, the glucuronide is covalently coupled to a lysine residue of the albumin, preferably as an acid amide.

In a more preferred embodiment of the conjugate of the present invention, the molar ratio of the drug-glucuronide to albumin is about 1:1.

Any corticosteroid that is pharmacologically active and, preferably, shows anti-inflammatory and/or anti-allergic properties is suitable for the purposes of the present invention. Preferred corticosteroids are dexamethasone, hydrocortisone and prednisolone.

The present invention relates to the use of the conjugate of the present invention for the preparation of a pharmaceutical composition. For administration the conjugate is preferably combined with suitable pharmaceutical carriers. Examples of suitable pharmaceutical carriers are well known in the art and include phosphate buffered saline solutions, water, emulsions, such as oil/water emulsions, various types of wetting agents, sterile solutions etc.. Such carriers can be formulated by conventional methods and can be administered to the subject at a suitable dose. Administration of the suitable compositions may be effected by different ways, e.g. by intravenous, intraperetoneal, subcutaneous, intramuscular, topical or intradermal administration. The route of administration, of course, depends on the nature of the disease and the kind of conjugate contained in the pharmaceutical composition. The dosage regimen will be determined by the attending physician and other clinical factors, such as plasma halflives of the conjugate. As is well known in the medical arts, dosages for any one patient depends on many factors, including the patient's size, body surface area, age, sex, the particular compound to be administered, time and route of administration, the kind and stage of the disease, general health and other drugs being administered concurrently.

The present invention also relates to various therapeutic uses of the conjugate of the present invention. Preferably, the conjugate of the present invention is used for the treatment of an inflammatory disease, preferably a rheumatic disease, uveitis, asthma, Morbus Crohn, lupus erythomathodes and psoriasis.

A further preferred therapeutic use of the conjugate of the present invention is the prevention/treatment of graft-vs.-host disease or tumor.

"Tumor" in the context of the present application means any neoplastic cell growth. They include cancer of the bladder, bone, bone marrow, brain, breast, cervix, gall bladder, gastrointestinal tract, kidney, liver, lung, muscle, ovary, pancreas, parathyroid, prostate, skin, salivary glands, spleen, testis, uterus, particularly breast.

The present invention is explained by the examples.

### Example 1

### Synthesis of a hydrocortisone-21-β-D-glucuronide-albumin conjugate and dexamethasone-21-β-D-glucuronide-albumin conjugate

### (A) Chemical synthesis of the corticosteroid-21-β-D-glucuronides

The synthesis of hydrocortisone-21-β-D-glucuronic acid (HCG) and dexamethasone-21-β-D-glucuronic acid (DMG) was carried out as described by Haeberlein, B. et al., Pharmac. Res. 10 (1993), 1553, except that after flash chromatography of the deprotected glucuronide a preparative HPLC purification was performed. Column: 250 x 10 mm Prosep C18-GOI-5µ + VS Latek [Latek, D-69214 Heidelberg, Germany], eluent: 0.1% COOH in H₂0 (A) 70%, CH₃CN (B) 30%; flow rate: 16 ml/min, 254 nm; retention time (RT) of HCG: 6.1 min. Eluent for DMG: 65% A, 35% B; RT: 5.6 min. The purified fraction of the glucuronides was controlled by analytical RP-18 HPLC (column: 150 x 4 mm Prosep C18-GOI-5µ Latek A [Latek, Heidelberg] and analysed by mass spectrometry. The eluent was removed by vacuum drying, the precipitate dissolved in water and lyophilized.

### (B) Synthesis of the hydrocortisone-glucuronide-albumin conjugate

19.8 mg (35.6 µMol) purified HCG and 50 mg (450 µMol) hydroxyl succinimide were dissolved in 1 ml dimethyl acetamide with addition of 1 ml dioxan. Then, 9 mg (71.4 µMol, 11 µl) diisopropyl carbodiimide were added and the mixture left for 24 h at room temperature. HPLC control of the formed HCG-succinimide-ester (HCG-SE) after hydrolysis: 100 µl (2 mg) lysine in 0.1 M NaHCO₃ were added to 2 µl of the reaction solution and allowed to react for 1 h at 50°C. 20 µl of the product were analyzed by HPLC; Column: 150 x 4 mm Prosep C18-GOI-5µ Latek, eluent: 70% A, 30% B, 1 ml/min, 254 nm.

2.0 g Human serum albumin (HSA) (from Octapharm,D-40764 Langenfeld, Germany) washed with 250 ml H₂0 via ultrafiltration (5 times), lyophilized) were dissolved in 20 ml of 0.1 M NaHCO₃ and added to 5 ml of CH₃CN. By use of a glass syringe fitted with a 0.2 µm filter (Schleicher + Schüll, D-37586 Passel, Germany), the HCG-SE solution was slowly added to the HSA solution under constant stirring and stirred for additional 60 min.. H₂0 was added to the solution to give a final volume of 50 ml. For analysis of the coupling reaction, 20 µl of the solution thus obtained were subjected to gel filtration HPLC (SEC); column: Zorbax GF 250, 250 x 9 mm, Agilent (Bischoff, D-71229 Leonberg, Germany); flow rate: 1 ml/min.; eluent: 180 mM sodium acetate, 20 mM sodium citrate, pH 7.5, 0.05% NaN₃, 254 nm. RT HSA: 9,48 min., HCG: 14,45 min.

To check for unbound glucuronides, 1 ml of the solution was taken, mixed with 1 ml of H₂O and subjected to ultrafiltration with Centricon C50 (Millipore GmbH, D-65760 Eschborn, Germany). 20 µl of the filtered solution were analyzed for unreacted glucuronide by RP-18 HPLC. The HSA solution was purified by ultrafiltration over Amicon YM membranes (4 times; by adding each time 200 ml physiological NaCl solution containing 160 mg/l gentamycine). The volumes of the filtered solutions were determined and the content of unbound HCG was determined by HPLC in the filtrates. The purified protein solution was adjusted to 100 mg/ml HSA. 5.3 mg HCG were present in the pooled filtrates, i.e., 14.5 mg HCG (with 19.8 mg used as initial amount) were coupled (73.2%), 0.725 mg HCG/ml to 100 mg HSA.

The structure of the hydrocortisone-glucuronide-albumin conjugate is shown in Figure 2A.

### (C) Synthesis of the dexamethasone-glucuronide-albumin conjugate

The synthesis of dexamethasone-21-β-glucuronide-HSA was essentially carried out as described for hydrocortisone-21-β-glucuronide-HSA except that HCG was replaced by DMG (16.6 mg; 29.2 µMol). The formation of the succinimid-ester was controlled with 2 mg serine in 100 µl NaHCO₃ at 45°. The eluent used for RP-18 HPLC was 65% A and 35% B. The reaction of DMG-succinimide ester was 100%. 1.5 g HSA were dissolved in 15 ml 0.1 M NaHCO₃ (plus 5 ml CH₃CN). The retention times of the products after SEC chromatography were 9.48 min. (for HSA) and 16.24 min. (for DMG). The protein solution was purified by ultrafiltration with Amicon YM membranes (5 times). The purified protein solution was adjusted to 100 mg/ml HSA. 6 mg of unbound DMG were present in the pooled filtrates, i.e., 10.6 mg DMG (with 16.6 mg used as initial amount) were coupled (64%), 0.7 mg DMG/ml to 100 mg HSA.

The structure of the dexamethasone-glucuronide-albumin conjugate is shown in Figure 2B.

### Example 2

### Biological effects of the conjugates of the present invention

### (A) Introduction

Thus, steroid glucuronides conjugated to HSA are degraded after endocytoses in the lysosomes to such an extent that the released steroid can diffuse from the lysosome and enter the nucleus through the action of receptors. From studies of modifications of natural corticosteroids it is known that alterations at particular positions of the molecule result in loss of its biological activity, e.g., position 3 and the hydroxyl group at position 21.

In order to enhance solubility in water and to improve renal excreation the steroids are glucuronidated in the body. These natural glucuronids are coupled via position 3 and cleavage by glucuronidases would result in a hydroxyl group and, thus, a molecule species showing reduced biological activity compared to the initial compound. By contrast, a glucuronide at position 21 is advantageous, since after cleavage by a glucuronidase the original molecule showing no loss of biological activity is present.

As illustrated in Example 1, steroid glucuronides were synthesized according to the method of Haeberlein et al., supra, with the modifications described below. The coupling of the glucuronide was performed via a Bolton-Hunter reaction with a lysine residue of the albumin to an acid amide. The coupling was carried out with a molar ratio of about 1:1 since higher loaded albumins become denatured, are immunogenic and are degraded in the liver.

In the experiments described below it could be shown that hydrocortisone-21-β-D-glucuronide-HSA conjugate is a substrate for glucuronidases as shown by the biological effects of the conjugate in human WEHI-7 cells. The cleavage of fluorescent methyl umbelliferon from methyl umbelliferon-glucuronide-HSA used as a model substrate in synovial fibroblasts and in conditioned medium of these fibroblasts could be demonstrated.

### (B) Determination of the effects of HC and HCG-HSA in cell culture

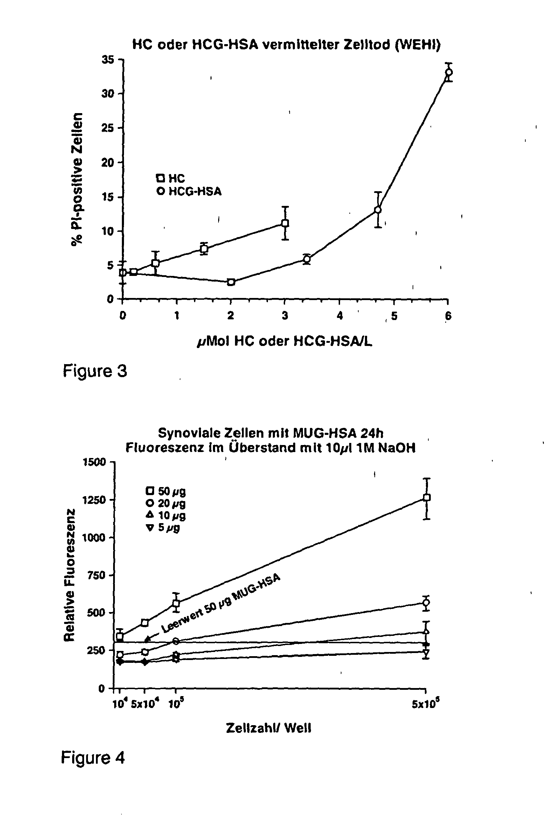

Cell death in glucocorticoid-sensitive WEHI-7 cells was induced by incubation with low molecular hydrocortisone (HC) and albumin-coupled hydrocortisone (hydrocortisone-21-β-D-glucuronide-HSA; HCG-HSA) over a period of 72 h. Different albumin concentrations were equalized by addition of free HSA. Dead cells were determined by fluorimetric analysis (staining with propidium iodide (PI)). It could be demonstrated that despite coupling to albumin HCG keeps its activity as a glucocorticoid. Since the albumin conjugate was taken up by the cells via endocytosis (whereas the free HC passes the cell membrane via diffusion) HCG-HSA has to be applied in a two-fold concentration for achieving the same effect. In vitro the hydrocortisone-HSA conjugate exerts its effect as a glucocorticoid by inducing a dose dependent cell death. The results are shown in Figure 3.

### (C) Release of a fluorescent aglycon from MUG-HSA in human synovial fibroblasts

Cleavage of methyl umbelliferon glucuronide-HSA (MUG-HSA) after incubation for 24 h in synovial fibroblasts (sFb) could be demonstrated. The results are shown in Figure 4. The amounts given in the Figure relate to 1 well and the methyl umbelliferon glucuronide within the HSA conjugate. After addition of 1 M NaOH methyl umbelliferon was fluorimetrically determined in the cell subernatant at 448 nm with an excitation at 360 nm. It could be demonstrated that sFb posses glucuronidase acitivity which cleaves MUG-HSA. Thus, despite its coupling to albumin MUG-HSA is a substrate for human synovial fribroblasts. MUG-(HSA) is stable in medium without cells.

## Claims

1. A pharmaceutical composition comprising a conjugate comprising (a) a pharmacologically active drug, (b) a glucuronic acid and (c) an albumin, wherein said pharmacologically active drug is a corticosteroid.

2. The pharmaceutical composition of claim 1, wherein said glucuronic acid is coupled at position 21 of the corticosteroid to form a glucuronide.

3. The pharmaceutical composition of claim 1 or 2, wherein said glucuronide is coupled to a lysine residue of the albumin as an acid amide.

4. The pharmaceutical composition of any one of claims 1 to 3, wherein the molar ratio of the drug-glucuronide to albumin is 1:1.

5. The pharmaceutical composition according to any one of claims 1 to 4, wherein said albumin is human serum albumin.

6. The pharmaceutical composition according to any one of claims 1 to 5, wherein said corticosteroid is dexamethasone, hydrocortisone or prednisolone.

7. Use of the conjugate of any one of claims 1 to 6 for the preparation of a pharmaceutical composition for the treatment of an inflammatory disease.

8. Use according to claim 7, wherein said inflammatory disease is a rheumatic disease, uveitis, asthma, Morbus Crohn, lupus erythomathodes, psoriasis.

9. Use of the conjugate of any one of claims 1 to 6 for the preparation of a pharmaceutical composition for the treatment or prevention of graft-versus-host disease or cancer.

10. Use according to claim 9, wherein said cancer is mammary carcinoma.

## Patentansprüche

1. Pharmazeutische Zusammensetzung umfassend ein Konjugat, das (a) ein pharmakologisch wirksames Arzneimittel, (b) eine Glucuronsäure und (c) ein Albumin umfasst, wobei das pharmakologisch wirksame Arzneimittel ein Kortikosteroid ist.

2. Pharmazeutische Zusammensetzung nach Anspruch 1, wobei die Glucuronsäure an die Position 21 des Kortikosteroids zur Bildung eines Glucuronids gekoppelt ist.

3. Pharmazeutische Zusammensetzung nach Anspruch 1 oder 2, wobei das Glucuronid an einen Lysinrest des Albumins als Säureamid gebunden ist.

4. Pharmazeutische Zusammensetzung nach einem der Ansprüche 1 bis 3, wobei das Molverhältnis des Arneimittel-Glucuronids zu Albumin 1:1 beträgt.

5. Pharmazeutische Zusammensetzung nach einem der Ansprüche 1 bis 4, wobei das Albumin humanes Serumalbumin ist.

6. Pharmazeutische Zusammensetzung nach einem der Ansprüche 1 bis 5, wobei das Kortikosteroid Dexamethason, Hydrokortison oder Prednisolon ist.

7. Verwendung des Konjugats nach einem der Ansprüche 1 bis 6 für die Herstellung einer pharmazeutischen Zusammensetzung für die Behandlung einer entzündlichen Erkrankung.

8. Verwendung nach Anspruch 7, wobei die entzündliche Erkrankung eine rheumatische Erkrankung, Uveitis, Asthma, Morbus Crohn, Lupus erythematodes, Psoriasis ist.

9. Verwendung des Konjugats nach einem der Ansprüche 1 bis 6 für die Herstellung einer pharmazeutischen Zusammensetzung für die Behandlung oder Prävention einer Transplantat-Wirt-Reaktion oder von Krebs.

10. Verwendung nach Anspruch 9, wobei der Krebs ein Mammakarzinom ist.

## Revendications

1. Composition pharmaceutique comprenant un conjugué comprenant (a) un médicament actif pharmacologiquement, (b) un acide gluconique et (c) une albumine, **caractérisée en ce que** le médicament actif pharmacologiquement est un corticostéroïde.

2. Composition pharmaceutique selon la revendication 1, **caractérisée en ce que** l'acide gluconique est couplé en position 21 de corticostéroïde pour former un glucuronide.

3. Composition pharmaceutique selon la revendication 1 ou 2, **caractérisée en ce que** le glucoronide est couplé à un résidu de lysine de l'albumine comme un amide acide.

4. Composition pharmaceutique selon l'une des revendications 1 à 3, **caractérisée en ce que** le ratio molaire du glucuronide médicamenteux par rapport à l'albumine est de 1 :1.

5. Composition pharmaceutique selon l'une des revendications 1 à 4, **caractérisée en ce que** ladite albumine est une albumine de sérum humain.

6. Composition pharmaceutique selon l'une des revendications 1 à 5, **caractérisée en ce que** le corticostéroïde est la dexaméthasone, l'hydrocortisone ou la prédnisolone.

7. Utilisation d'un conjugué selon l'une des revendications 1 à 6 pour la préparation d'une composition pharmaceutique pour le traitement d'une maladie inflammatoire.

8. Utilisation selon la revendication 7, **caractérisée en ce que** la maladie inflammatoire est constituée par une maladie rhumatismale, uveite, asthme, maladie de Crohn, lupus érythémateux, psoriasis.

9. Utilisation d'un conjugué selon l'une des revendications 1 à 6 pour la préparation d'une composition pharmaceutique pour le traitement ou la prévention de la maladie d'une greffe par rapport au sujet ou le cancer.

10. Utilisation selon la revendication 9, **caractérisé en ce que** le cancer est le carcinome mammaire.
